# EUROPÄISCHE PATENTSCHRIFT

(11) **EP 2 510 905 B1**
(45) Veröffentlichungstag und Bekanntmachung des Hinweises auf die Patenterteilung: **11.06.2014**
(21) Anmeldenummer: 12002422.9
(22) Anmeldetag: 03.04.2012
(51) Int. Cl.: A61F 2/52

(54) **Brustprothese**
Breast prosthesis
Prothèse mammaire

(30) Priorität: 11.04.2011 DE 202011005141 U
(43) Veröffentlichungstag der Anmeldung: 17.10.2012
(73) Patentinhaber: Amoena Medizin-Orthopädie-Technik GmbH, 83064 Raubling (DE)
(72) Erfinder: Reusch, Michaela, 83395 Freilassing (DE); Wild, Helmut, 83071 Stephanskirchen (DE)
(74) Vertreter: Laufhütte, Dieter

(56) Entgegenhaltungen:
- EP-A1- 0 125 400
- EP-A1- 1 232 733
- DE-A1-102008 050 362
- DE-C1- 4 421 516
- DE-U1- 8 701 925
- DE-U1- 9 315 935

## Beschreibung

Die Erfindung betrifft eine Brustprothese zum Ersatz einer operativ wenigstens teilweise entfernten weiblichen Brust.

Gattungsgemäße Brustprothesen sind aus dem Stand der Technik bekannt.

In den letzten Jahren hat sich eine neue Technik bei der operativen Entfernung und der anschließenden Rekonstruktion von beschädigtem Brustgewebe etabliert. So ist es möglich, das komplette Brustgewebe zu entfernen, wenn möglich über den Warzenhof, so daß die *"Brusthülle"* erhalten bleibt. Sodann wird ein Expander unterhalb des Brustmuskels eingesetzt, um eine spätere Implantatversorgung vorzubereiten. Der Brustmuskel braucht jedoch eine gewisse Zeit, um sich zu dehnen. Daher wird der Expander typischerweise anfangs mit einem geringen Volumen eingesetzt, und dieses Volumen erst über einen Zeitraum von Wochen oder Monaten sukzessive erhöht, bis das Volumen des einzusetzenden Implantats erreicht ist. Nach einer gewissen Haltedauer wird der Expander anschließend gegen ein permanentes Implantat ausgetauscht.

Eine Brustprothese, die begleitend zur obengenannten Technik während der Regenerations- und Expansionsphase getragen wird, muß sich so an eine sukzessive veränderte Größe der verbleibenden Brust angepaßt werden.

Zu diesem Zweck ist es aus dem Stand der Technik bekannt, anstelle einer bekannten Silikonprothese eine Prothese zur Erstversorgung einzusetzen.

Die Anmelderin vertreibt eine solche unter der Bezeichnung "Priform". Dieses Produkt besteht im Wesentlichen aus einer formgebenden Stützschale aus Kunststoff, die in einem Stoffteil aufgenommen ist, und weist an der Rückseite einen Hohlraum auf, in dem ein Füllmaterial aufgenommen werden kann. Die Stützschale kann jedoch weder die Tasteigenschaften noch die Bewegungseigenschaften einer natürlichen Brust wiedergegeben.

Aus der EP 0 808 615 A1 ist eine Brustprothese aus einem weichelastischen Kunststoffkörper bekannt, an dessen Rückseite ein elastisches Stoffteil angeschweißt ist. In einem Hohlraum zwischen dem Stoffteil und dem Kunststoffkörper kann Füllmaterial aufgenommen sein, das der Anpassung an eine sich verändernde Größe der verbliebenen Brust dienen kann. Jedoch ist das Stoffteil untrennbar mit dem Kunststoffkörper verbunden und kann zum Waschen nicht abgenommen werden, was aus hygienischen Gesichtspunkten nachteilig sein kann.

Aus der EP 1 232 733 A1 ist eine Brustprothese bekannt, welche im Wesentlichen aus in Kunststofffolien eingeschweißten, der Brustform nachgebildeten Körpern aus einer Zweikomponenten-Silikoncautschuk-Masse besteht. Um den Tragekomfort zu verbessern wird diese Prothese derart ausgebildet, dass sie zumindest in ihrer dem Körper zugewandten Seite wenigstens teilweise aus einem PCM-Material, welches eine Phasenumwandlungstemperatur im Bereich der Körpertemperatur aufweist, besteht.

Aus der EP 0 125 400 A1 ist eine Brustprothese bekannt, in deren Aushöhlung ein Formstück eingesetzt ist, welches eine schalenförmige weiche Schicht aufweist.

Die Brustprothese weist einen Kunststoffkörper und einen Polsterkörper auf, welche in einer Stofftasche angeordnet und gegebenenfalls aus dieser entnommen werden können. Der Polsterkörper kann hierbei als Wattebausch ausgeführt werden.

Die DE 93 15 935 U1 offenbart eine Brustprothese mit einer harten Schicht, die schalenförmig ausgebildet ist. Die harte Schicht ist zwischen einer schalenförmigen mittelharten Schicht und einer schalenförmigen weichen Schicht eingebettet. An der Rückseite dieser bekannten Brustprothese befindet sich eine Füllkammer, die zur Aufnahme eines Füllmaterials geeignet ist.

Aufgabe der Erfindung ist es daher, ausgehend von einer aus der DE 93 15 935 U1 bekannten Brustprothese, eine verbesserte Brustprothese bereitzustellen, die an eine sich verändernde Narbenstruktur beziehungsweise Größe der verbliebenen Brust angepaßt werden kann und welche einen verbesserten Tragekomfort bietet sowie den hohen hygienischen Anforderungen einer solchen Prothese Rechnung trägt.

Diese Aufgabe wird mit einer Brustprothese gemäß Anspruch 1 gelöst. Vorteilhafte Ausgestaltungen ergeben sich aus den Unteransprüchen.

So ist erfindungsgemäß eine Brustprothese mit einer Stützschale vorgesehen, die in einem Stoffbezug aufgenommen ist. An der Rückseite der Brustprothese befindet sich wenigstens eine Füllkammer zur Aufnahme eines Füllmaterials. Die Brustprothese weist eine vor der Stützschale angeordnete Silikonschale auf, die entnehmbar in dem Stoffüberzug aufgenommen ist.

Ferner wirkt die erfindungsgemäße Brustprothese durch das Vorhandensein einer Stützschale als Schutz für das schmerzempfindliche frisch operierte Gewebe. Die Silikonschale kann einfach aus dem Stoffüberzug entnommen werden, um diese zu waschen. Somit ist die erfindungsgemäße Brustprothese aus hygienischen Gesichtspunkten vorteilhaft. Die Stützschale ist fest mit dem Stoffüberzug zumindest an der Rückseite der Stützschale verbunden.

Die erfindungsgemäße Brustprothese kann die genannten Bestandteile aufweisen oder daraus bestehen. Vorzugsweise sind die Silikonschale und/oder die Stützschale vollständig von dem Stoffüberzug umgeben. Als Vorderseite der erfindungsgemäßen Brustprothese wird die dem Träger abgewandte Seite der Brustprothese bezeichnet. Als Hinterseite wird die dem Träger zugewandte Seite bezeichnet.

Durch die Aufnahme von variablen Mengen eines Füllmaterials in der wenigstens einen Füllkammer an der Rückseite kann die Brustprothese während einem eingangs beschriebenen Rekonstruktionsverlauf, der mit einer sukzessive veränderten Größe der verbliebenen Brust einhergeht, stufenlos angepaßt werden. Das Volumen der Brustprothese wird dabei vorzugsweise so reguliert, daß das Gesamtvolumen der operierten Seite (verbleibende Brust + Prothese) stets der gesunden Brust angeglichen wird.

Durch die vorgelagerte Anordnung der Silikonschale vor der Stützschale wird eine weiche und natürliche Außenerscheinung erreicht. Das natürliche äußere Erscheinungsbild und die natürlichen Tasteigenschaften erlauben neben dem Einsatz als Prothese zur Erstversorgung auch einen darüber hinausgehenden Einsatz als multifunktionelle Leichtprothese. Durch die Kombination einer flachen Silikonschale mit einer Stützschale kann das Gewicht gegenüber Standardprothesen um etwa 50 % verringert werden, was selbst gegenüber handelsüblichen Leichtprothesen eine zusätzliche Gewichtseinsparung bringt.

So findet eine erfindungsgemäße Brustprothese hauptsächlich während der Expander-Behandlung als Vorbereitung für ein Implantat oder als Leichtprothese Verwendung.

Die Stützschale ist vorzugsweise flächig und schalenförmig und besitzt eine konvex geformte Vorderseite. Sie kann eine über deren Fläche hinweg konstante Stärke beziehungsweise Dicke aufweisen, wobei sie sich gegebenenfalls in den Randbereichen verjüngen kann.

Die Silikonschale ist vorzugsweise ebenfalls flächig und schalenförmig und besitzt eine konvex geformte Vorderseite, welche der natürlichen Form der weiblichen Brust nachempfunden ist. Die Rückseite ist vorzugsweise konkav ausgebildet, und kann das Gegenstück zur konvexen Vorderseite der Stützschale bilden. Vorzugsweise ist die Stärke beziehungsweise Dicke der Silikonschale im zentralen Bereich größer als in den Randbereichen. In den Randbereichen kann die Silikonschale sehr dünn sein und gegebenenfalls nur die Stärke einer Kunststofffolie aufweisen, die die Hülle der Silikonschale bilden kann. Die Silikonschale ist wenigstens in deren zentralem Bereich typischerweise dicker als die Stützschale, wobei die Stärke der Silikonschale auch mindestens das Doppelte oder auch mindestens das Dreifache der Stärke der Stützschale betragen kann. Die Stärke der Silikonschale ist jedoch vorteilhafterweise auch auf einen gewissen Maximalwert beschränkt, und beträgt beispielsweise weniger als das zehnfache, und weiter vorzugsweise weniger als das fünffache der Stärke der Stützschale.

In einer Ausführungsform erfolgt die feste Verbindung der Stützschale mit dem Stoffüberzug vorzugsweise in einem ringförmigen und flächigen Randbereich an der Rückseite der Stützschale. Der Randbereich umgibt typischerweise eine zentrale Fläche an der Rückseite der Stützschale und erstreckt sich über eine gewisse Stärke bis zum Rand der Stützschale. Geeignete Arten der Verbindung des Stoffüberzugs mit der Stützschale umfassen unter anderem Verschweißen, Verkleben und Vernähen.

Alternativ kann die Stützschale aus dem Stoffüberzug entnommen werden. In diesem Zusammenhang ist beispielsweise eine halbfeste Verbindung der Stützschale mit dem Stoffüberzug denkbar, beispielsweise durch einen Klettverschluß.

In einer Ausführungsform ist die Füllkammer im zentralen Bereich an der Rückseite der Stützschale angeordnet.

In einer Ausführungsform grenzen die Stützschale und die Silikonschale innerhalb des Stoffüberzugs direkt aneinander an, sodaß sich zwischen der Stützschale und der Silikonschale kein Element des Stoffüberzugs befindet. Alternativ können die Stützschale und die Silikonschale jedoch auch in verschiedenen Aufnahmekammern des Stoffüberzugs aufgenommen sein.

In einer Ausführungsform ist die Silikonschale der Stützschale vollflächig vorgelagert. Dabei soll die rückseitige Oberfläche der Silikonschale im Wesentlichen der vorderseitigen Oberfläche der Stützschale entsprechen.

In einer Ausführungsform weist der Stoffüberzug wenigstens zwei flächige Stoffteile auf, die entlang ihres gemeinsamen Randes verbunden sind. Der Stoffüberzug kann auch aus diesen Stoffteilen bestehen. Im Bereich ihres gemeinsamen Randes können diese Stoffteile beispielsweise vernäht, verschweißt oder verklebt sein.

In einer Ausführungsform weist der Stoffüberzug eine Öffnung zur Entnahme der Silikonschale auf. Bei der Öffnung kann es sich beispielsweise um einen Öffnungsschlitz handelt, der an der Unterseite der Prothese angebracht ist. Diese Öffnung beziehungsweise dieser Öffnungsschlitz können verschließbar oder nicht verschließbar sein. Mögliche Verschlußmechanismen sind zum Beispiel Klettverschluß, Reißverschluß, Haken und Ösen, Druckknöpfe oder sich überlagernde und gegebenenfalls gespannte Abschnitte des Stoffüberzugs. Die Öffnung kann sich, falls vorhanden, im Verbindungsbereich mehrerer den Stoffüberzug bildender Stoffteile befinden beziehungsweise an deren Verbindungslinie verlaufen.

Im Falle einer entnehmbaren Anordnung der Stützschale kann dieselbe Öffnung auch zur Entnahme der Stützschale dienen. Alternativ kann bei einer derartigen Anordnung eine weitere Öffnung vorgesehen sein, die in einer Ausführungsform ebenfalls eines oder mehrere der im Zusammenhang mit der Öffnung zur Entnahme der Silikonschale beschriebenen Eigenschaften aufweisen kann.

In einer Ausführungsform besteht der Stoffüberzug aus einem vorzugsweise elastischen Kunstfasergewebe. Alternativ dazu kann auch eine Naturfaser wie beispielsweise Baumwolle eingesetzt werden. Bevorzugt ist die Fertigung aus Mikrofasergewebe, das insbesondere Mikrofasern aus Polyester und/oder Polyamid aufweist. Derartige Klimastoffe unterstützen die Abfuhr von Feuchtigkeit von der Haut.

In einer Ausführungsform sind die Vorderseite und/oder die Rückseite des Stoffüberzugs der erfindungsgemäßen Brustprothese gespannt. Dies gilt insbesondere dann, wenn die Silikonschale neben der Stützschale im Stoffüberzug aufgenommen ist. Diese Spannung kann auf der Fertigungsseite durch das Tiefziehen beziehungsweise Molden eines elastischen Stoffüberzugs im Zuge der Formgebung der Stützschale und/oder der Silikonschale erreicht werden.

In einer Ausführungsform ist die Füllkammer wenigstens teilweise durch den Stoffüberzug und/oder die Rückseite der Stützschale begrenzt. Beispielsweise kann die Füllkammer als Zwischenraum zwischen der Rückseite der Stützschale und dem Stoffüberzug ausgebildet sein beziehungsweise durch die Rückseite der Stützschale und den Stoffüberzug definiert sein. Vorzugsweise liegt die Füllkammer daher direkt an der Rückseite der Stützschale an, im Regelfall in deren zentralem Bereich. Die Füllkammer kann sich beispielsweise über denjenigen Bereich der Rückseite der Stützschale erstrecken, in dem die Stützschale, falls zutreffend, nicht fest mit dem Stoffüberzug verbunden ist. Im Bereich der Kammer kann der Stoffüberzug gespannt ausgebildet sein, oder auch lose ausgebildet sein.

In einer Ausführungsform weist die Füllkammer eine Öffnung zur Entnahme des Füllmaterials auf. Diese kann sich beispielsweise innerhalb des Stoffüberzugs an der Rückseite der Füllkammer befinden, oder im Verbindungsbereich des Stoffüberzugs und der Stützschale. Vorzugsweise handelt es sich bei der Öffnung um einen Öffnungsschlitz, der verschließbar oder auch nicht verschließbar sein kann. Geeignete Verschlußmechanismen umfassen beispielsweise sich überlagernde und gegebenenfalls gespannte Abschnitte des Stoffüberzugs in den Randbereichen der Öffnung oder Klettverschlüsse.

In einer Ausführungsform ist in der Füllkammer ein Füllmaterial aufgenommen. Geeignete Füllmaterialien umfassen wattenförmige Stoffe, wie beispielsweise Faserwatte oder Fadenwatte, worunter in der vorliegenden Erfindung ein loses Volumengefüge von Fasern beziehungsweise Fäden verstanden wird. Alternativ oder zusätzlich zum wattenartigen Füllmaterial können in der Füllkammer ein oder mehrere Polsterkörper aufgenommen sein, worunter eine Gewebehülle oder Folie verstanden wird, die wiederum ein Füllmaterial umgibt. Als Füllmaterialien eignet sich wiederum Watte. Geeignete Materialien zum Bereitstellen der Fäden oder Fasern für die Watte beziehungsweise für eine Gewebehülle umfassen Baumwollfasern oder Kunstfasern, insbesondere Mikrofasern wie Polyester oder Polyamid. Ebenfalls geeignete Füllstoffe sind geschäumte Kunststoffe, wie zum Beispiel Schaumstoffflocken oder Styropor, sowie Hohlkugeln oder Gele.

In einer Ausführungsform weist die Brustprothese mehrere Füllkammern auf, beispielsweise zwei oder drei individuelle Füllkammern. So kann in manchen Fällen (Teiloperationen, misslungene Wiederaufbauten, etc.) eine bessere Formanpassung an die verbleibende, unter der Prothese liegende Brust erreicht werden. Dabei kann vorgesehen sein, daß anwendungsspezifisch nur eine, mehrere oder auch alle individuellen Füllkammern befüllt werden. In einer Ausführungsform können die individuellen Füllkammern seitlich nebeneinander angeordnet sein. Auch Anordnungen als übereinanderliegende Streifen beziehungsweise Ebenen, als konzentrische Kreise, im Schachbrettmuster, oder individuell angepaßt sind denkbar und von der Erfindung umfaßt. Gegebenenfalls können die individuellen Kammern durch Nähte beziehungsweise Schweißnähte voneinander getrennt sein. Jede Kammer kann so als Zwischenraum zwischen der Rückseite der Stützschale und dem Stoffüberzug ausgebildet sein. Jede individuelle Kammer kann eine Öffnung zur Entnahme des Füllmaterials aufweisen, wie sie im Zusammenhang mit einer einzigen Kammer näher beschrieben wurde.

In einer Ausführungsform besteht die Stützschale aus Kunststoff. Vorzugsweise handelt es sich dabei um einen weichen beziehungsweise elastischen Kunststoff, der jedoch formstabil, d. h. nicht plastisch verformbar sein sollte. Bevorzugt ist beispielsweise der Einsatz weicher Polyurethanschäume oder Abstandsgewirke, wie diese auch typischerweise in den Schalen eines BH Verwendung finden.

In einer Ausführungsform ist die Silikonschale ein weichelastischer Kunststoffkörper, der vorzugsweise aus einer in einer Kunststofffolie eingeschweißten Silikonmasse besteht. Bei der Kunststofffolie kann es sich um eine PU-Folie handeln. Beispiele für geeignete Silikonmassen umfassen unter anderem eine Zwei-Komponenten-Silikon-Kautschukmasse.

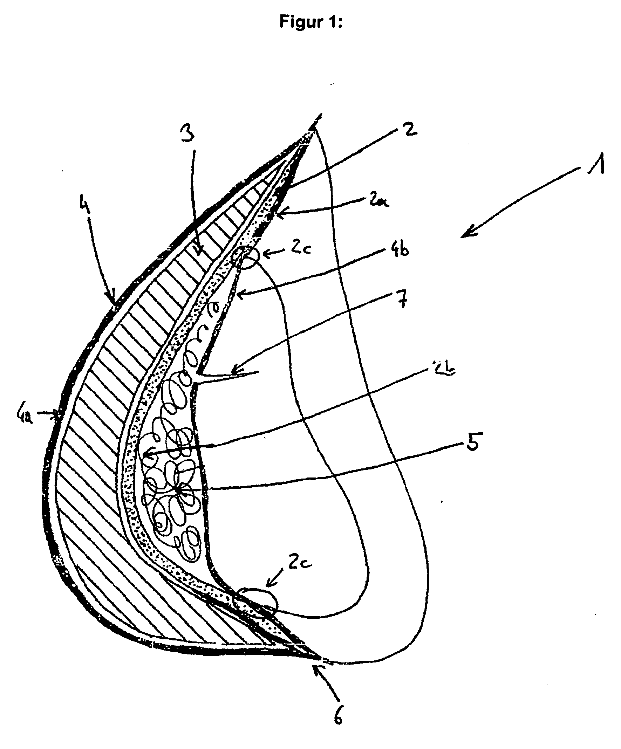

Weitere Einzelheiten und Vorteile der Erfindung ergeben sich aus den nachfolgend anhand der Figur beschriebenen Ausführungsbeispielen.

Die einzige Figur zeigt eine Darstellung einer erfindungsgemäßen Brustprothese im Querschnitt.

Die Brustprothese wird generell mit dem Bezugszeichen 1 bezeichnet. Sie weist eine Stützschale 2 aus einem weichen PU-Schaum auf, die eine konstante Dicke besitzt und schalenförmige ist. In den Endbereichen weist diese Schale eine etwas geringere Stärke auf.

An der Vorderseite der Stützschale 2 schließt vollflächig eine weichelastische Silikonschale 3 an, die aus einer in eine PU-Folie eingeschweißten Zwei-Komponenten-Silikon-Kautschukmasse besteht. Die Silikonschale 3 hat an deren Außenseite eine der natürlichen Form der Brust nachempfundene Gestalt. Die konkave Innenseite ist der Krümmung der Außenseite der Stützschale 2 angepaßt. Die Stärke der Silikonschale ist im zentralen Bereich größer als in den Randbereichen, wo sie spitz zusammenläuft.

Stützschale 2 und Silikonschale 3 sind in einem Stoffüberzug 4 aufgenommen, welcher aus flächigen Stoffteilen 4a und 4b besteht, die entlang ihres gemeinsamen Randes verbunden sind.

Die Stützschale 2 ist entlang der Innenkante 2c eines ringförmigen Randbereichs 2a an deren Rückseite mit dem Stoffüberzug 4 vernäht.

Im zentralen Bereich 2b an der Rückseite der Stützschale befindet sich eine Füllkammer 5, welche mit Watte gefüllt ist. Diese Watte kann durch den Öffnungsschlitz 7 im Stoffüberzug eingebracht und entnommen werden. Die Kammer 5 wird durch die Rückseite der Stützschale 2 und durch den Stoffüberzug 4 definiert.

Im Verbindungsbereich der Stoffteile 4a und 4b an der Unterseite der Brustprothese befindet sich eine Öffnung 6, durch die die Silikonschale 3 bei Bedarf entnommen werden kann. Dadurch ergibt sich nicht nur ein hygienischer Vorteil beim Waschen der Brustprothese, sondern auch die Möglichkeit, diese mit unterschiedlich geformten Silikonschalen 3 auszustatten.

Zusammenfassend ergibt sich, daß eine erfindungsgemäße Brustprothese in einfacher Weise an eine sich verändernde Narbenstruktur oder Größe der verbliebenen Brust angepaßt werden kann, ein geringes Gewicht aufweist und gleichzeitig gesteigerten ästhetischen Anforderungen gerecht werden kann.

## Patentansprüche

1. Brustprothese (1) mit einer Stützschale (2) sowie einer vor der Stützschale (2) angeordneten Silikonschale (3), wobei sich an der Rückseite der Brustprothese (1) eine oder mehrere Füllkammern (5) zur Aufnahme eines Füllmaterials befinden,
**dadurch gekennzeichnet, daß**
die Stützschale (2) in einem Stoffüberzug (4) aufnehmbar ist, wobei die Silikonschale (3) entnehmbar in dem Stoffüberzug (4) aufgenommen ist, und wobei die Stützschale (2) fest mit dem Stoffüberzug (4) zumindest an der Rückseite der Stützschale verbunden ist.

2. Brustprothese nach Anspruch 1, **dadurch gekennzeichnet, daß** die Stützschale (2) in einem ringförmigen und flächigen Randbereich (2a) an der Rückseite der Stützschale (2) verbunden ist.

3. Brustprothese nach Anspruch 1 oder 2, **dadurch gekennzeichnet, daß** die Silikonschale (3) sowie der mit Füllstoff (5) aufgepolsterte Stoffüberzug (4) separat tragbar und waschbar sind.

4. Brustprothese nach einem der vorhergehenden Ansprüche, **dadurch gekennzeichnet, daß** die Füllkammer(n) (5) im zentralen Bereich (2b) an der Rückseite der Stützschale (2) angeordnet ist/sind.

5. Brustprothese nach einem der vorhergehenden Ansprüche, **dadurch gekennzeichnet, daß** die Stützschale (2) und die Silikonschale (3) innerhalb des Stoffüberzugs (4) direkt aneinander angrenzen.

6. Brustprothese nach einem der vorhergehenden Ansprüche, **dadurch gekennzeichnet, daß** die Silikonschale (3) der Stützschale (2) vollflächig vorgelagert ist.

7. Brustprothese nach einem der vorhergehenden Ansprüche, **dadurch gekennzeichnet, daß** der Stoffüberzug (4) wenigstens zwei flächige Stoffteile (4a, 4b) aufweist oder daraus besteht, die entlang ihres gemeinsamen Randes verbunden sind.

8. Brustprothese nach einem der vorhergehenden Ansprüche, **dadurch gekennzeichnet, daß** der Stoffüberzug (4) eine Öffnung (6) zur Entnahme der Silikonschale (3) aufweist, wobei die Öffnung (6) des Stoffüberzugs (4) zur Entnahme der Silikonschale (3) vorzugsweise verschließbar ist.

9. Brustprothese nach einem der vorhergehenden Ansprüche, **dadurch gekennzeichnet, daß** der Verschluß der Öffnung (6) des Stoffüberzugs (4) zur Entnahme der Silikonschale (3) mit Klettverschluß, Reißverschluß, Haken oder Ösen, Druckknöpfen oder sich überlagernden und gegebenenfalls gespannten Abschnitten des Stoffüberzugs versehen ist.

10. Brustprothese nach einem der vorhergehenden Ansprüche, **dadurch gekennzeichnet, daß** die Füllkammer(n) (5) wenigstens teilweise durch den Stoffüberzug (4) und/oder die Rückseite der Stützschale (2) begrenzt ist/sind, daß die Füllkammer(n) (5) vorzugsweise als Zwischenraum zwischen der Rückseite der Stützschale (2) und dem Stoffüberzug (4) ausgebildet ist/sind und daß die Füllkammer(n) (5) besonders bevorzugt eine Öffnung (7) zur Entnahme des Füllmaterials aufweist/aufweisen.

11. Brustprothese nach einem der vorhergehenden Ansprüche, **dadurch gekennzeichnet, daß** in der/den Füllkammer(n) (5) ein Füllmaterial aufgenommen ist, vorzugsweise Watte.

12. Brustprothese nach einem der vorhergehenden Ansprüche, **dadurch gekennzeichnet, daß** die Stützschale (2) aus Kunststoff besteht, vorzugsweise einem weichen beziehungsweise elastischen, jedoch formstabilen Kunststoff.

13. Brustprothese nach einem der vorhergehenden Ansprüche, **dadurch gekennzeichnet, daß** die Silikonschale (3) ein weichelastischer Kunststoffkörper ist und vorzugsweise aus einer in einer Kunststoffolie eingeschweißten Silikonmasse besteht.

14. Brustprothese nach dem vorhergehenden Anspruch, **dadurch gekennzeichnet, daß** das Füllmaterial vorzugsweise aus Watte oder geschäumten Kunststoffen, wie zum Beispiel Schaumstoffflocken oder Styropor, sowie aus Hohlkugeln oder Gelen besteht.

15. Brustprothese nach einem der vorhergehenden Ansprüche, **dadurch gekennzeichnet, daß** die Stützschale (2) aus Kunststoff besteht, vorzugsweise einem weichen beziehungsweise elastischen, jedoch formstabilen Kunststoff, oder daß die Stützschale (2) aus weichem Polyurethanschaum oder Abstandsgewirk besteht.

## Claims

1. Breast prosthesis (1) having a support shell (2) as well as a silicon shell (3) arranged in front of the support shell (2), wherein one or more filling chambers (5) are located at the rear of the breast prosthesis (1) to receive a filler material,
**characterised in that**,
the support shell (2) can be received in a fabric cover (4), wherein the silicon shell (3) can be received detachably in the fabric cover (4) and wherein the support shell (2) is connected fixedly to the fabric cover (4) at least at the rear of the support shell.

2. Breast prosthesis according to claim 1, **characterised in that** the support shell (2) is connected in an annular and flat edge region (2a) at the rear of the support shell (2).

3. Breast prosthesis according to claim 1 or 2, **characterised in that** the silicon shell (3) and the fabric cover (4) stuffed with the filler material (5) can be carried and washed separately.

4. Breast prosthesis according to one of the preceding claims, **characterised in that** the filling chamber(s) (5) is/are arranged in the central region (2b) at the rear of the support shell (2).

5. Breast prosthesis according to one of the preceding claims, **characterised in that** the support shell (2) and the silicon shell (3) directly adjoin each other within the fabric cover (4).

6. Breast prosthesis according to one of the preceding claims, **characterised in that** the silicon shell (3) is located completely in front of the support shell (2).

7. Breast prosthesis according to one of the preceding claims, **characterised in that** the fabric cover (4) has or consists of at least two flat fabric parts (4a, 4b) that are connected along their mutual edge.

8. Breast prosthesis according to one of the preceding claims, **characterised in that** the fabric cover (4) has an opening (6) for detaching the silicon shell (3), wherein the opening (6) of the fabric cover (4) can preferably be closed for detaching the silicon shell (3).

9. Breast prosthesis according to one of the preceding claims, **characterised in that** the closing of the opening (6) of the fabric cover (4) for detaching the silicon shell (3) has Velcro, a zip, hooks or loops, buttons or overlapping and potentially taut sections of the fabric cover added to it.

10. Breast prosthesis according to one of the preceding claims, **characterised in that** the filling chamber(s) (5) is/are at least partially bordered by the fabric cover (4) and/or the rear of the support shell (2), the filling chamber(s) (5) is/are preferably formed as an intermediate space between the rear of the support shell (2) and the fabric cover (4) and the filling chamber(s) (5) particularly preferably has/have an opening (7) for the detachment of the filler material.

11. Breast prosthesis according to one of the preceding claims, **characterised in that** a filler material, preferably cotton wool, is received in the filling chamber(s) (5).

12. Breast prosthesis according to one of the preceding claims, **characterised in that** the support shell (2) consists of plastic, preferably a weak or elastic yet dimensionally stable plastic.

13. Breast prosthesis according to one of the preceding claims, **characterised in that** the silicon shell (3) is a weakly elastic plastic body and preferably consists of a silicon mass shrink-wrapped in a plastic film.

14. Breast prosthesis according to the preceding claim, **characterised in that** the filler material preferably consists of cotton wool or foamed plastics, such as foam flakes or Styrofoam, as well as of hollow spheres or gels.

15. Breast prosthesis according to one of the preceding claims, **characterised in that** the support shell (2) consists of plastic, preferably a weak or elastic yet dimensionally stable plastic, or the support shell (2) consists of a weak polyurethane foam or woven mesh.

## Revendications

1. Prothèse mammaire (1) comportant une coque de soutien (2), ainsi qu'une coque de silicone (3) disposée en avant de la coque de soutien (2), une ou plusieurs chambres de remplissage (5) destinées à recevoir un matériau de remplissage se trouvant contre la face arrière de la prothèse mammaire (1), **caractérisée en ce que** la coque de soutien (2) peut être logée dans une enveloppe (4), la coque de silicone (3) étant logée d'une manière amovible dans l'enveloppe (4), et la coque de soutien (2) étant fermement liée à l'enveloppe (4), au moins contre la face arrière de la coque de soutien.

2. Prothèse mammaire selon la revendication 1, **caractérisée en ce que** la coque de soutien (2) est, dans une zone marginale annulaire et plate (2a), liée à la face arrière de la coque de soutien (2).

3. Prothèse mammaire selon la revendication 1 ou 2, **caractérisée en ce que** la coque de silicone (3), ainsi que l'enveloppe (4) rembourrée du matériau de remplissage (5), peuvent être portées et lavées séparément.

4. Prothèse mammaire selon l'une des revendications précédentes, **caractérisée en ce que** la ou les chambres de remplissage (5) sont disposées dans la zone centrale (2b) contre la face arrière de la coque de soutien (2).

5. Prothèse mammaire selon l'une des revendications précédentes, **caractérisée en ce que** la coque de soutien (2) et la coque de silicone (3) sont immédiatement adjacentes l'une à l'autre à l'intérieur de l'enveloppe (4).

6. Prothèse mammaire selon l'une des revendications précédentes, **caractérisée en ce que** la coque de silicone (3) se trouve sur toute sa surface en avant de la coque de soutien (2).

7. Prothèse mammaire selon l'une des revendications précédentes, **caractérisée en ce que** l'enveloppe (4) comprend au moins deux parties plates (4a, 4b) ou en est constituée, qui sont reliées le long de leur bord commun.

8. Prothèse mammaire selon l'une des revendications précédentes, **caractérisée en ce que** l'enveloppe (4) comprend une ouverture (6) pour prélever la coque de silicone (3), l'ouverture (6) de l'enveloppe (4), destinée à prélever la coque de silicone (3), pouvant de préférence être refermée.

9. Prothèse mammaire selon l'une des revendications précédentes, **caractérisée en ce que** la fermeture de l'ouverture (6) de l'enveloppe (4) pour le prélèvement de la coque de silicone (3) est pourvue d'une fermeture auto-agrippante, d'une fermeture à glissière, de crochets ou d'oeillets, de boutons-poussoirs, ou de segments de l'enveloppe en chevauchement et éventuellement tendus.

10. Prothèse mammaire selon l'une des revendications précédentes, **caractérisée en ce que** la ou les chambres de remplissage (5) sont au moins en partie délimitées par l'enveloppe (4) et/ou la face arrière de la coque de soutien (2), que la ou les chambres de remplissage (5) sont de préférence configurées comme des espaces intermédiaires entre la face arrière de la coque de soutien (2) et l'enveloppe (4), et que la ou les chambres de remplissage (5) présentent d'une manière particulièrement préférée une ouverture (7) pour prélever le matériau de remplissage.

11. Prothèse mammaire selon l'une des revendications précédentes, **caractérisée en ce qu'**un matériau de remplissage, de préférence du coton, est logé dans la ou les chambres de remplissage (5).

12. Prothèse mammaire selon l'une des revendications précédentes, **caractérisée en ce que** la coque de soutien (2) est constituée d'un matériau plastique, de préférence d'un matériau plastique souple, ou élastique, mais à stabilité de forme.

13. Prothèse mammaire selon l'une des revendications précédentes, **caractérisée en ce que** la coque de silicone (3) est un corps en un matériau plastique élastique souple, et est de préférence constituée d'une masse de silicone insérée par soudage dans une feuille plastique.

14. Prothèse mammaire selon l'une des revendications précédentes, **caractérisée en ce que** le matériau de remplissage est de préférence constitué de coton ou de plastiques expansés, tels que par exemple des flocs de matériau mousse ou du polystyrène, ainsi que de billes creuses ou de gels.

15. Prothèse mammaire selon l'une des revendications précédentes, **caractérisée en ce que** la coque de soutien (2) est constituée d'un matériau plastique, de préférence d'un matériau plastique souple ou élastique, mais à stabilité de forme, ou que la coque de soutien (2) est constituée d'une mousse de polyuréthanne souple ou d'un tissu tricoté d'écartement.
